(19) Europäisches Patentamt
European Patent Office
Office européen des brevets

(11) Veröffentlichungsnummer: **0 217 747 B1**

# (12) EUROPÄISCHE PATENTSCHRIFT

(45) Veröffentlichungstag der Patentschrift: **21.11.91**

(51) Int. Cl.[5]: **C07D 271/10**, C07D 285/12, A01N 43/82

(21) Anmeldenummer: **86810419.1**

(22) Anmeldetag: **24.09.86**

Die Akte enthält technische Angaben, die nach dem Eingang der Anmeldung eingereicht wurden und die nicht in dieser Patentschrift enthalten sind.

## (54) Nematizide Mittel.

(30) Priorität: **30.09.85 CH 4214/85**

(43) Veröffentlichungstag der Anmeldung:
**08.04.87 Patentblatt 87/15**

(45) Bekanntmachung des Hinweises auf die Patenterteilung:
**21.11.91 Patentblatt 91/47**

(84) Benannte Vertragsstaaten:
**AT BE CH DE FR GB IT LI LU NL**

(56) Entgegenhaltungen:
**EP-A- 0 036 711**
**FR-A- 2 211 008**
**US-A- 2 744 908**
**US-A- 3 513 172**
**US-A- 3 770 754**
**US-A- 4 454 147**

(73) Patentinhaber: **CIBA-GEIGY AG**
**Klybeckstrasse 141**
**CH-4002 Basel(CH)**

(72) Erfinder: **Beriger, Ernst, Dr.**
**Grabenmattweg 29**
**CH-4123 Allschwil(CH)**

EP 0 217 747 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte Alkylmercaptophenyl-1,3,4-oxadiazol- bzw. -1,3,4-Thiadiazol-Derivate, deren Herstellung sowie nematizide Mittel, die als Wirkstoff mindestens eine dieser Verbindungen enthalten. Die Erfindung betrifft ferner die Verwendung der neuen Wirkstoffe und Mittel zur Bekämpfung von Nematoden, insbesondere von pflanzenschädigenden Nematoden.

Die erfindungsgemässen Verbindungen fallen unter folgende allgemeine Formeln

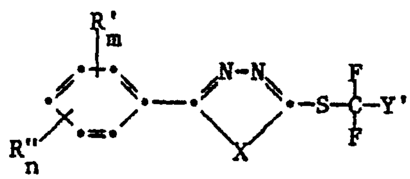

(Ia),

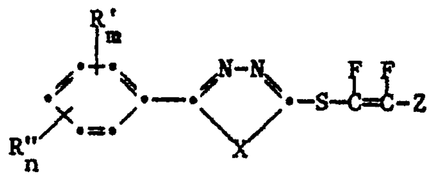

(Ib),

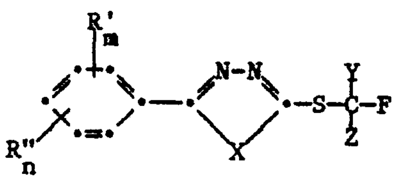

(I*),

in welchen

- R' $C_1$-$C_5$-Alkyl, $C_1$-$C_5$-Alkoxy, Halogen oder Trifluormethyl bedeutet.
- R" $C_1$-$C_5$-Alkyl oder Halogen darstellt,
- m für 1 bis 3 und n für 0 oder 1 stehen,
- X Sauerstoff oder Schwefel bedeutet,
- Y Wasserstoff, Fluor, Trifluormethyl oder die Gruppe HC(F)Z und
- Y' Wasserstoff oder die Gruppe $CHF_2$ darstellen und
- Z für Fluor oder Trifluormethyl steht, und

schliessen die Säureadditionssalze mit organischen oder anorganischen Säuren mit ein.

Unter Alkyl sind als selbständiger Rest sowie als Teil einer anderen Gruppe, wie Alkoxy, gerad- und verzweigtkettige Alkylgruppen zu verstehen. Dazu zählen die Methyl-, die Ethyl- sowie die Isomeren der Propyl-, der Butyl- und der Pentylgruppe, darunter insbesondere Methyl, Ethyl, n-Propyl, Isopropyl, Isobutyl oder n-Pentyl. Halogen steht für Fluor, Chlor, Brom oder Jod, vorzugsweise für Fluor oder Chlor.

Beispiele salzbildender Säuren sind unter den anorganischen Säuren Halogenwasserstoffsäuren wie Fluorwasserstoffsäure, Chlorwasserstoffsäure, Bromwasserstoffsäure oder Jodwasserstoffsäure sowie ferner Schwefelsäure, Phosphorsäure, phosphorige Säure, Salpetersäure und unter den organischen Säuren Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Es sind bereits Oxadiazol- und Thiadiazol-Derivate bekannt, die als nematizid wirksam beschrieben sind. So sind in der US-Patentschrift 3,770,754 solche Verbindungen mit einer 1,2,4-Stellung der Heteroatome offenbart, während in der US-Patentschrift 4,454,147 1,3,4-Thiadiazol-Derivate beschrieben sind, bei denen im Vergleich mit den erfindungsgemässen Verbindungen der Heterocyclus anstelle der Mercapto-Gruppen durch ein Chloratom substituiert ist. Diese bekannten Verbindungen haben bisher als Nematizide die in der Praxis an sie gestellten Ansprüche nicht in vollen Umfang befriedigen können.

Durch die Bereitstellung der erfindungsgemässen Verbindungen der Formeln Ia, Ib, und I* ist es nun gelungen einen wertvollen Beitrag zur Bekämpfung der beträchtliche landwirtschaftliche Schäden an Pflanzgut verursachenden Pflanzennematoden zu leisten. Dadurch können Ernteeinbussen bei Kulturpflanzen wie z.B. Kartoffeln, Getreide, Rüben, Raps, Kohl und Gemüse sowie Schäden in Baumschulen und im Zierpflanzenbau nachhaltig eingedämmt werden. Die erfindungsgemässen Verbindungen zeichnen sich dabei insbesondere in der wirkungsvollen Bekämpfung von wurzelparasitären Bodennematoden wie z.B. solche der Gattungen Heterodera und Globodera (zystenbildende Nematoden), Meloidogyne (Wurzelgallennematoden) sowie der Gattungen Pratylenchus, Paratylenchus, Longidorus, Trichodorus und Xiphinema aus. Ferner lassen sich mit den erfindungsgemässen Wirkstoffen die Nematodengattungen Ditylenchus (Stengelparasiten) Aphelenchoides (Blattnematoden) und Anguina (Blütennematoden) wirkungsvoll bekämpfen.

Mit den Wirkstoffen der Formeln Ia, Ib und I* lassen sich vorzugsweise besonders schädliche Nematodenspecies der Gattung Meloidogyne, wie z.B. Meloidogyne incognita, der Gattung Heterodera, wie z.B. Heterodera glycines (Sojabohnen-Zystennematode), und ferner der Gattung Globodera, wie z.B. Globodera rostochienis (Kartoffelzystennematode) sowie der Gattung Radopholus, eine Bananenpflanzen schädigende endoparasitäre Wurzelnematode, erfolgreich bekämpfen.

Zur Bekämpfung der Pflanzennematoden und zur Gesunderhaltung des Pflanzgutes können die neuen Wirkstoffe kurativ, präventiv oder systemisch eingesetzt werden. Dabei entfalten sie eine breit gefächerte Aktivität gegen die verschiedenen Nematodenspecies und werden somit den Erfordernissen der Praxis gerecht. Die nematizide Wirkungsweise der erfindungsgemässen Verbindungen wird durch eine ihnen zueigene geringe Phytotoxizität in vorteilhafter Weise begleitet, womit der allgemein wünschenswerten Verminderung der Umweltbelastung in besonderem Masse Rechnung getragen wird.

Im Rahmen der vorliegenden Erfindung sind folgende Verbindungen der Formeln Ia, Ib und I* bevorzugt:

1) Verbindungen, in denen R' $C_1$-$C_2$-Alkyl, $C_1$-$C_2$-Alkoxy, Halogen oder Trifluormethyl und R" $C_1$-$C_4$-Alkyl oder Halogen bedeuten und m für 1 oder 2 steht.

2) Verbindungen, in denen R' Methyl, Methoxy, Fluor oder Chlor und R" Fluor oder Chlor bedeuten, Y Wasserstoff und Z Fluor darstellen.

Bevorzugte Einzelverbindungen

2-Difluormethylthio-5-(4'-methoxyphenyl)-1,3,4-oxadiazol;
2-Difluormethylthio-5-(4'-methoxyphenyl)-1,3,4-thiadiazol;
2-Difluormethylthio-5-(2'-fluorphenyl)-1,3,4-thiadiazol;
2-Difluormethylthio-5-(4'-chlorphenyl)-1,3,4-thiadiazol;
2-Difluormethylthio-5-(2'-methylphenyl)-1,3,4-thiadiazol;
2-Difluormethylthio-5-(2',4'-dichlorphenyl)-1,3,4-thiadiazol;
2-Difluormethylthio-5-(2'6'-difluorphenyl)-1,3,4-thiadiazol;
2-Difluormethylthio-5-(4'-fluorphenyl)-1,3,4,-thiadiazol;
2-Difluormethylthio-5-(3'-chlorphenyl)-1,3,4,-thiadiazol.

Die Verbindungen der Formeln Ia, Ib und I*, werden erfindungsgemäss hergestellt, indem man

a) in einer Kondensationsreaktion eine Verbindung der Formel IIa

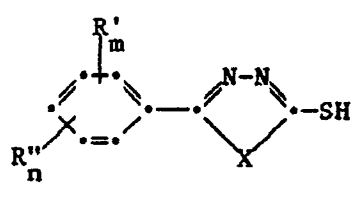

(IIa),

oder eine Verbindung der Formel IIb

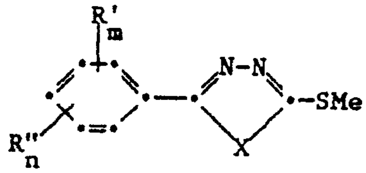

(IIb)

mit einer Verbindung der Formel IIIa

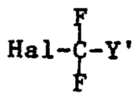

(IIIa)

oder mit einer Verbindung der Formel III*

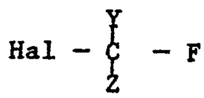

(III*)

in einem inerten Lösungsmittel oder Lösungsmittel-Gemisch bei erhöhter Temperatur gegebenefalls in Gegenwart eine Katalysators und gegebenenfalls bei erhöhtem Druck umsetzt, wobei die Umsetzung einer Verbindung der Formel IIa in Gegenwart einer Base verläuft, oder indem man

b) in einer Anlagerungsreaktion einer Verbindung der Formel IIa

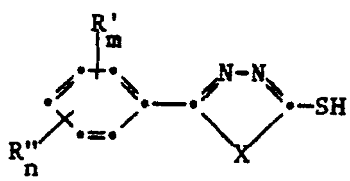

(IIa),

mit einer Verbindung der Formel IIIb

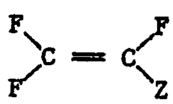

(IIIb)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ia*

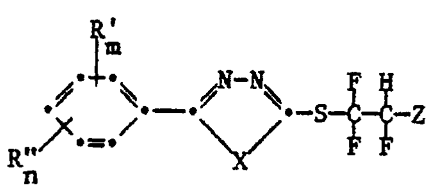

(Ia*),

oder zu einer Verbindung der Formel Ib

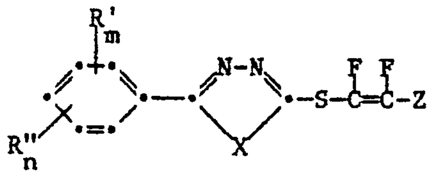 (Ib),

führt.

In den vorstehend genannten Formeln IIa, IIb, Ia*, Ib, III*, IIIa und IIIb bedeuten Me ein Alkalimetall oder Ammonium, Hal Halogen, vorzugsweise Chlor, Brom oder Jod, während R', R", m, n, Y, Y' und Z die unter den Formeln Ia, Ib und I* angegebenen Bedeutungen besitzen.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert. -Butylmethylether usw.), Anisol, Diozan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie Aceton, Diethylketon, Methylethylketon sowie Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), sowie Oxide, Hydroxide, Carbonate und Hydrogencarbonate von Alkyli- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriumethylat.

Die Zugabe katalytischer Mengen einer Kronenethers, wie z.B. 18-Krone-6 oder 15-Krone-5 wirkt sich in den Herstellungsverfahren günstig auf den Reaktionsablauf aus. Ferner hat sich für den gleichen Zweck die katalytische Verwendung von Tetraalkylaminsalzen, z.B. Tetraalkylaminochloride oder -bromide, vorzugsweise Tetra-n-butylaminobromid, als vorteilhaft erwiesen. Darüber hinaus sind Alkalijodide, vorzugsweise Kaliumjodid, als Katalysatoren vorteilhaft einsetzbar.

In den Herstellungsverfahren betragen die Reaktionstemperaturen 10° bis 90° C, vorzugsweise 30° C bis 80° C. Für die Druckverhältnisse während des Reaktionsablaufs sind 1 bis 20 bar, vorzugsweise 6 bis 14 bar, massgebend.

Die Erfindung betrifft auch Mittel, zur Bekämpfung von pflanzenschädigenden Nematoden sowie zur präventiven Verhütung des Nematodenbefalls von Pflanzgut, welche Wirkstoffe der Formel Ia, Ib oder I* enthalten.

Darüber hinaus schliesst die vorliegende Erfindung zusätzlich die Herstellung nematizider Mittel ein, die gekennzeichnet ist durch das innige Vermischen von Aktivsubstanzen der Formel Ia, Ib oder I* mit einer oder mehreren hierin beschriebenen Trägerstoffen und Hilfsingredienzen. Eingeschlossen ist auch ein Verfahren zur Behandlung von Pflanzen, das durch die Applikation der Verbindungen der Formel Ia, Ib oder I* bzw. der neuen Mittel charakterisiert ist.

Die Ausgangsverbindungen der Formeln IIa und IIb sind teils bekannt, teils neu. Die neuen Verbindungen der genannten Formeln sind Zwischenprodukte zur Herstellung wertvoller nematozider Wirkstoffe (siehe Tabelle 0) und bilden damit einen Bestandteil der vorliegenden Erfindung.

Die Ausgangsverbindungen der Formeln IIa und IIb können nach bekannten Methoden wie folgt hergestellt werden:

a) Die 2-Mercapto-1,3,4-oxadiazole sind gewinnbar durch Zugabe von Schwefelkohlenstoff zu einer Lösung des entsprechend substituierten Benzhydrazids in alkoholisch-wässrigem Kaliumhydroxid und Erhitzen des Reaktionsgemisches während einiger Stunden. Als Lösungsmittel werden dabei Alkohole wie z.B. Ethylalkohol oder n-Amylalkohol verwendet. Durch Ansäuern der gebildeten Kaliumsalze werden die freien Mercapto-Verbindungen erhalten [vgl. J.Am.Chem.Soc. 78, 4975-4978 (1956)].

b) Die 2-Mercapto-1,3,4-thiadiazole sind erhältlich durch Behandlung des entsprechend substituierten Benzoyl-kaliumdithiocarbazats mit konzentrierter Schwefelsäure bei -5° bis 10° C [vgl. J.prakt. Chem. 93, 49 (1916); J.Org.Chem. 23, 1021 (1958); J.Heterocycl. Chem. 19, 542-544 (1982)].

Ein bezvorzugtes Verfahren für den Einsatz eines Wirkstoffs der Formel Ia, Ib oder I* bzw. eines nematiziden Mittels, das mindestens einen dieser Wirkstoffe enthält, ist das Einbringen in den Erdboden. Dabei wird der Standort der Pflanzen mit einer flüssigen oder festen Zubereitung behandelt.

Die Verbindungen der Formel Ia, Ib und I* können aber auch auf Samenkörner aufgebracht werden (Beizung/Coating), indem man die Körner entweder in einer flüssigen Zubereitung des Wirkstoffs tränkt oder sie mit einer festen Zubereitung beschichtet. Darüber hinaus sind in besonderen Fällen weitere Applikationsarten möglich, so z.B. die gezielte Behandlung der Pflanzenstengel oder Knospen.

Wirkstoffe der Formel Ia, Ib oder I* werden üblicherweise in Form von formulierten Zusammensetzungen verwendet und können gleichzeitig oder nacheinander mit weiteren Wirkstoffen auf die zu behandelnde Fläche oder Pflanze gegeben werden. Diese weiteren Wirkstoffe können auch andere in der Landwirtschaft angewendete Mittel umfassen, die in ihrer Nutzanwendung der Produktionssteigerung durch Förderung des Nutzpflanzenwachstums dienen, wie Düngemittel, Herbizide, Insektizide, Fungizide, Molluskizide u.a.,oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlich oder regenerierten mineralischen Stoffen, Lösungs-, Dispergier-, Netz-, Haft-, Verdickungs-, Binde- oder Düngemitteln.

Die Verbindungen der Formel Ia, Ib oder I* werden in unveränderter Form oder vorzugsweise zusammen mit den in der Formulierungstechnik üblichen Hilfsmitteln eingesetzt. Sie werden z.B. zu Emulsionskonzentraten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Verstäuben, Verstreuen oder Giessen werden gleich wie die Art der Mittel den angestrebten Zielen und den gegebenen Verhältnissen entsprechend gewählt. Günstige Aufwandmengen liegen im allgemeinen bei 500 g bis 6 kg Aktivsubstanz (AS) je ha; bevorzugt 1 bis 4 kg AS/ha.

Die Formulierungen, d.h. die den Wirkstoff der Formel Ia, Ib oder I* und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenfalls mit oberflächenaktiven Substanzen (Tensiden).

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon,Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Substanzen kommen je nach Art des zu formulierenden Wirkstoffes der Formel Ia, Ib oder I* nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. Das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens eines Alkylrest mit 8 bis 22 C-Atomen enthalten und als weitere Substituenten niedrige gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die agrochemischen Zubereitungen enthalten in der Regel 0,1 bis 99 Gew.-%, insbesondere 0,1 bis 95 Gew.-%, Wirkstoff der Formel Ia, Ib oder I*, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken.

1. Herstellungsbeispiele

Beispiel 1.1: Herstellung von

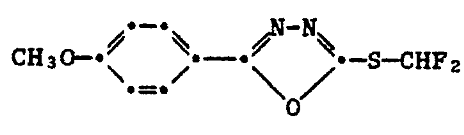

2-Difluormethylthio-5-(4'-methoxyphenyl)-1,3,4-oxadiazol

a) Zu einer Lösung von 4,5 g Kaliumhydroxid in 18 ml Wasser gibt man unter Rühren 10,2 g 2-Mercapto-5-(4'-methoxyphenyl)-1,3,4-oxadiazol und 100 ml Dioxan. Nach Zugabe von 0,2 g Kaliumjodid und 2 Kristallen 18-Krone-6 erwärmt man die Mischung auf 50-60 °C und leitet unter gutem Rühren während 6 Stunden einen schwachen Strom von Chlordifluormethan ein. Anschliessend dampft man die Reaktionsmischung im Vakuum ein. Der Eindampfrückstand wird in Methylenchlorid aufgenommen und nacheinander mit Wasser und 1-normaler Natronlauge ausgewaschen. Nach dem Abdampfen des Lösungsmittels erhält man die Titelverbindung (aus Cyclohexan umkristallisiert) als farblose Kristalle vom Schmelzpunkt 95-97 °C.

b) Zu einer Mischung von 3,2 g Kaliumhydroxid und 0,5 g Kaliumjodid in 40 ml Wasser und 200 ml Dioxan in einem Autoklav werden 29,5 g Kaliumsalz des 2-Mercapto-5-(4'-methoxyphenyl)-1,3,4-oxadiazol gegeben und gut verrührt. Man presst 51,9 g Chlordifluormethan hinein und erwärmt die Mischung

unter Rühren während 18 Stunden auf 45-50 °C (10-12 bar). Nach Abkühlen und Entspannung des Druckes giesst man das Reaktionsgemisch auf 500 ml Eiswasser. Die Kristalle werden abgesaugt und getrocknet. Man erhält die Titelverbindung als schwach gelblich gefärbte Kristalle vom Schmelzpunkt 93-96 °C.

Beispiel 1.2: Herstellung von

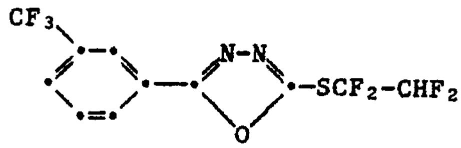

2-(1,1,2,2,-Tetrafluorethylthio)-5-(3'-trifluormethylphenyl)-1,3,4-oxadiazol

In einem Autoklaven werden 60 ml Dimethylformamid und 7,38 g 2-Mercapto-5-(3'-trifluormethylphenyl)-1,3,4-oxadiazol (Smp. 154-155 °) und 0,7 g Kaliumhydroxid verrührt. Man presst 22 g Tetrafluorethylen hinein und erwärmt die Reaktionsmischung während 19 Stunden auf 60 °C ($10\text{-}12 \cdot 10^5$ Pa). Nach Abkühlung und Entspannung des Druckes giesst man die Lösung auf Eiswasser, nimmt das Produkt in Chloroform auf, wäscht die Chloroformlösung mit 1-normaler Natronlauge durch und entfernt das Lösungsmittel im Vakuum. Als Rückstand erhält man die Titelverbindung als Oel mit dem Brechungsindex $n_D^{22}$ 1,4926.

Beispiel 1.3: Herstellung von

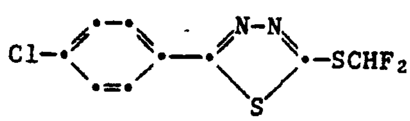

2-Difluormethylthio-5-(4'-chlorphenyl)-1,3,4,-thiadiazol

Zu einer Lösung von 2,4 g Kaliumhydroxid in 10 ml Wasser gibt man 6,9 g 2-Mercapto-5-(4'-chlorphenyl)-1,3,4-thiadiazol und 60 ml Dioxan. Nach Zugabe von 0,2 g Kaliumjodid und 2 Kristallen Tetrabutylammoniumbromid erwärmt man die Mischung auf 40 ° und leitet unter Rühren während 4 Stunden einen schwachen Strom Chlordifluormethan ein. Anschliessend dampft man die Reaktionsmischung im Vakuum ein. Der Eindampfrückstand wird in Methylenchlorid aufgenommen und nacheinander mit Wasser und 1-normaler Natronlauge gewaschen. Nach dem Abdampfen des Lösungsmittel erhält man die Titelverbindung (aus Isopropanol umkristallisiert) als farblose Kristalle vom Schmelzpunkt 74-76 °C.

Beispiel 1.4: Herstellung von

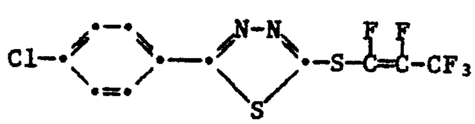

2-[1,2,3,3,3-Pentafluor-prop(1)enylthio]-5-(4-chlorphenyl)-1,3,4-thiadiazol

In einem Autoklaven werden 60 ml Dimethylformamid und 6,85 g 5-(4-Chlorphenyl)-2-mercapto-1,3,4-thiadiazol und 0,7 g Kaliumhydroxid verrührt. Man presst 20 g Hexafluorpropylen hinein und erwärmt die Reaktionsmischung während 19 Stunden auf 60 °C ($10\text{-}12 \cdot 10^5$ Pa). Nach Abkühlung und Entspannung des Druckes giesst man die Lösung auf Eiswasser, nimmt das Produkt in Chloroform auf, wäscht die Chloroformlösung mit 1-normaler Natronlauge durch und entfernt das Lösungsmittel im Vakuum. Als

Rückstand erhält man die Titelverbindung in Kristallform mit einem Schmelzpunkt von 69-70 °C.

Analog der vorstehenden Herstellungsbeispiele sowie der vorbeschriebenen Verfahren lassen sich folgende erfindungsgemässe Verbindungen herstellen. Die nachfolgend aufgeführten Verbindungen dienen der Illustration der vorliegenden Erfindung und stellen keine Begrenzung derselben dar.

Tabelle 0

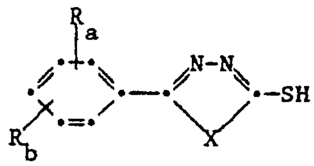

| Verb Nr. | $R_a$ | $R_b$ | X | Physikal. Daten [Smp. 0°C] |
|---|---|---|---|---|
| 0.1 | 3-$CF_3$ | H | O | 154-155 |
| 0.2 | 2-F | H | O | 174-176 |
| 0.3 | 2-$CH_3$ | 3-$CH_3$ | O | 170-172 |
| 0.4 | 2-$CH_3$ | 4-Cl | O | 196-198 |
| 0.5 | 3-$CH_3$ | H | O | 158-160 |
| 0.6 | 3-$CH_3$ | 5-$CH_3$ | O | 235-238 |
| 0.7 | 3-$OCH_3$ | H | O | 165-167 |
| 0.8 | 3-$CH_3$ | 4-$CH_3$ | O | 191-193 |
| 0.9 | 2-$CH_3$ | 5-$CH_3$ | O | 227-229 |
| 0.10 | 2-$OCH_3$ | H | O | 235-238 |
| 0.11 | 3-$CF_3$ | H | S | 173-176 |
| 0.12 | 2-Cl | 4-Cl | S | 228-230 |
| 0.13 | 2-F | H | S | 197-199 |
| 0.14 | 2-$CH_3$ | H | S | 164-166 |
| 0.15 | 4-$CH_3$ | H | S | 198 |
| 0.16 | 3-$CH_3$ | 5-$CH_3$ | S | 237-240 |
| 0.17 | 3-$OCH_3$ | H | S | 207-209 |

Tabelle 0 (Fortsetzung):

| Verb Nr. | $R_a$ | $R_b$ | X | Physikal. Daten [Smp. 0°C] |
|---|---|---|---|---|
| 0.18 | 2-$CH_3$ | 5-$CH_3$ | S | 195 |
| 0.19 | 2-$OCH_3$ | H | S | 220-223 |
| 0.20 | 3-$OCH_3$ | 4-$OCH_3$ | S | 244-246 |
| 0.21 | 4-$C_2H_5O$ | H | S | 203-206 |
| 0.22 | 2-F | 6-F | S | 190 |
| 0.23 | 4-t-$C_4H_9$ | H | S | 187-189° |
| 0.24 | 2-J | H | S | 176-178° |
| 0.25 | 2-Cl | 5-Cl | S | 223-225° |
| 0.26 | 3-Cl | 4-Cl | S | 226-228° |
| 0.27 | 4-$CF_3$ | H | S | 167-170° |
| 0.28 | 4-F | H | S | 210-212° |
| 0.29 | 3-Cl | 5-Cl | S | 239-241° |
| 0.30 | 2-$CH_3$ | 4-Cl | S | 212-215° |
| 0.31 | 3-$CH_3$ | 4-Cl | S | 213-216° |

Tabelle 1

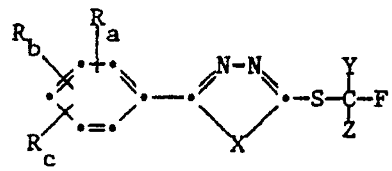

| Verb. Nr. | $R_a$ | $R_b$ | $R_c$ | X | Z | Y | Physikalische Konstante |
|---|---|---|---|---|---|---|---|
| 1.1 | H | 4-$OCH_3$ | H | O | F | H | Smp. 95-97°C |
| 1.2 | 3-$CF_3$ | H | H | O | F | $CHF_2$ | $n_D^{22}$ 1,4926 |
| 1.3 | H | 4-Cl | H | S | F | H | Smp. 74-76°C |
| 1.4 | H | 4-$CH_3$ | H | O | F | H | Smp. 104-106°C |
| 1.5 | 2-$CH_3$ | H | 6-$CH_3$ | O | F | H | $n_D^{22}$ 1,5550 |
| 1.6 | 3-$CH_3$ | 4-$CH_3$ | H | O | F | H | $n_D^{20}$ 1,5617 |
| 1.7 | 3-$OCH_3$ | H | H | O | F | H | $n_D^{22}$ 1,5528 |
| 1.8 | 2-$OCH_3$ | H | H | O | F | H | Harz |
| 1.9 | 3-$OCH_3$ | 4-$OCH_3$ | 5-$OCH_3$ | O | F | H | $n_D^{22}$ 1,5339 |
| 1.10 | 2-$CH_3$ | H | 5-$CH_3$ | O | F | H | $n_D^{22}$ 1,5146 |
| 1.11 | 2-$CH_3$ | 3-$CH_3$ | H | O | F | H | $n_D^{22}$ 1,5653° |
| 1.12 | 3-$CH_3$ | H | H | O | F | H | Smp. 47-50°C |
| 1.13 | 3-$CH_3$ | H | 5-$CH_3$ | O | F | H | Smp. 44-46°C |
| 1.14 | 3-$CF_3$ | H | H | O | F | H | $n_D^{22}$ 1,5188 |
| 1.15 | H | 4-Cl | H | O | F | H | Smp. 76-78°C |
| 1.16 | 3-$CH_3$ | H | 5-$CH_3$ | O | F | $CHF_2$ | $n_D^{22}$ 1,5290 |
| 1.17 | 3-$CH_3$ | H | H | S | F | H | Harz |
| 1.18 | H | 4-$OCH_3$ | H | S | F | H | Smp. 94-96° |
| 1.19 | 2-F | H | H | S | F | H | Smp. 45-47° |
| 1.20 | 3-$OCH_3$ | H | H | S | F | H | Smp. 38-41°C |
| 1.21 | 2-$CH_3$ | H | 5-$CH_3$ | S | F | H | Smp. 77-79°C |

Tabelle 1 (Fortsetzung):

| Verb. Nr. | $R_a$ | $R_b$ | $R_c$ | X | Z | Y | Physikalische Konstante |
|---|---|---|---|---|---|---|---|
| 1.22 | 3-$CF_3$ | H | H | S | F | H | $n_D^{22}$ 1,5568 |
| 1.23 | H | 4-$CH_3$ | H | S | F | H | Smp. 88-91°C |
| 1.24 | 3-$CH_3$ | H | 5-$CH_3$ | S | F | H | $n_D^{22}$ 1,6037 |
| 1.25 | 2-$OCH_3$ | H | H | S | F | H | Smp. 63-65°C |
| 1.26 | 2-$CH_3$ | H | H | S | F | H | $n_D^{22}$ 1,6094 |
| 1.27 | 2-Cl | 4-Cl | H | S | F | H | Smp. 79-81°C |
| 1.28 | 2-F | H | 6-F | S | F | H | Smp. 41-42°C |
| 1.29 | 3-$OCH_3$ | 4-$OCH_3$ | H | S | F | H | Smp. 129-131°C |
| 1.30 | 3-$CH_3$ | 4-$CH_3$ | H | S | F | H | Smp. 42-44°C |
| 1.31 | H | 4-$OC_2H_5$ | H | S | F | H | Smp. 94-96°C |
| 1.32 | H | 4-t-$C_4H_9$ | H | S | F | H | Smp. 50-52°C |
| 1.33 | 2-J | H | H | S | F | H | $n_D^{22}$ 1,6668 |
| 1.34 | 3-Cl | H | H | S | F | H | Smp.41-43°C |
| 1.35 | 2-Cl | H | 5-Cl | S | F | H | Smp.85-87°C |
| 1.36 | 3-Cl | 4-Cl | H | S | F | H | Smp.56-59°C |
| 1.37 | 2-Cl | H | H | S | F | H | $n_D^{23}$ 1,6217 |
| 1.38 | 2-$CH_3$ | 4-Cl | H | S | F | H | Smp.51-52°C |
| 1.39 | 2-Cl | H | 6-Cl | S | F | H | $n_D^{25}$ 1,5923 |
| 1.40 | H | 4-$CF_3$ | H | S | F | H | |
| 1.41 | H | 4-$OCH_3$ | H | S | F | F | |
| 1.42 | H | 4-$OCH_3$ | H | S | F | $CF_3$ | |
| 1.43 | H | 4-$OCH_3$ | H | S | $CF_3$ | $CF_3$ | |

Tabelle 1 (Fortsetzung):

| Verb. Nr. | $R_a$ | $R_b$ | $R_c$ | X | Z | Y | Physikalische Konstante |
|---|---|---|---|---|---|---|---|
| 1.44 | H | 4-$OCH_3$ | H | S | F | $HC(F)CF_3$ | |
| 1.45 | H | 4-$OCH_3$ | H | S | $CF_3$ | $CHF_2$ | |
| 1.46 | H | 4-$CH_3$ | H | S | F | F | |
| 1.47 | H | 4-$CH_3$ | H | S | F | $CF_3$ | |
| 1.48 | H | 4-$CH_3$ | H | S | $CF_3$ | $CF_3$ | |
| 1.49 | H | 4-$CH_3$ | H | S | F | $HC(F)CF_3$ | |
| 1.50 | H | 4-$CH_3$ | H | S | $CF_3$ | $CHF_2$ | |
| 1.51 | 3-$CH_3$ | H | H | S | F | F | |
| 1.52 | 3-$CH_3$ | H | H | S | F | $CF_3$ | |
| 1.53 | 3-$CH_3$ | H | H | S | $CF_3$ | $CF_3$ | |
| 1.54 | 3-$CH_3$ | H | H | S | F | $HC(F)CF_3$ | |
| 1.55 | 3-$CH_3$ | H | H | S | $CF_3$ | $CHF_2$ | |
| 1.56 | H | 4-Cl | H | S | F | F | |
| 1.57 | H | 4-Cl | H | S | F | $CF_3$ | |
| 1.58 | H | 4-Cl | H | S | $CF_3$ | $CF_3$ | |
| 1.59 | H | 4-Cl | H | S | F | $HC(F)CF_3$ | |
| 1.60 | H | 4-Cl | H | S | $CF_3$ | $CHF_2$ | |
| 1.61 | 2-F | H | 6-F | S | F | F | |
| 1.62 | 2-F | H | 6-F | S | F | $HC(F)CF_3$ | |
| 1.63 | 2-F | H | 6-F | S | $CF_3$ | $CF_3$ | |
| 1.64 | 2-F | H | 6-F | S | F | $CF_3$ | |
| 1.65 | 2-F | H | 6-F | S | $CF_3$ | $CHF_2$ | |

Tabelle 1 (Fortsetzung):

| Verb. Nr. | $R_a$ | $R_b$ | $R_c$ | X | Z | Y | Physikalische Konstante |
|---|---|---|---|---|---|---|---|
| 1.66 | 2-F | H | 6-F | O | F | H | |
| 1.67 | 2-F | H | 6-F | O | F | F | |
| 1.68 | H | 4-F | H | S | F | H | Smp. 59-60°C |
| 1.69 | 3-Cl | H | 5-Cl | S | F | H | Smp. 68-70°C |
| 1.70 | 3-$CH_3$ | 4-Cl | H | S | F | H | Smp. 42-44°C |
| 1.71 | 3-Br | H | H | S | F | H | Smp. 43-45°C |
| 1.72 | 3-F | H | H | S | F | H | Smp. 37-38°C |

Tabelle 2:

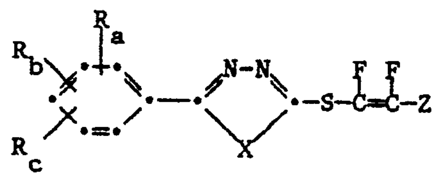

| Verb. No. | $R_a$ | $R_b$ | $R_c$ | X | Z | Physikal. Konstante |
|---|---|---|---|---|---|---|
| 2.1 | H | 4-Cl | H | S | $CF_3$ | Smp.69-70°C |
| 2.2 | 2-F | H | 6-F | S | $CF_3$ | |
| 2.3 | H | 4-F | H | S | F | |
| 2.4 | 3-Cl | H | H | S | $CF_3$ | |
| 2.5 | H | 4-$CH_3O$ | H | O | $CF_3$ | |
| 2.6 | 3-F | H | H | S | F | |
| 2.7 | 2-$CH_3$ | H | H | O | $CF_3$ | |
| 2.8 | 2-F | H | 6-F | S | F | |

2. Formulierungsbeispiele für flüssige Wirkstoffe der Formel Ia, Ib oder I* (% = Gewichtsprozent)

| 2.1 Emulsions-Konzentrate | a) | b) | c) |
|---|---|---|---|
| Wirkstoff aus den Tabellen 1 und 2 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten

Konzentration hergestellt werden.

| 2.2 Lösungen | a) | b) | c) | d) |
|---|---|---|---|---|
| Wirkstoff aus den Tabellen 1 und 2 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190° C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus den Tabellen 1 und 2 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1 und 2 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

<u>Formulierungsbeispiele für feste Wirkstoffe der Formel Ia, Ib oder I* (% = Gewichtsprozent)</u>

| 2.5 Spritzpulver | a) | b) | c) |
|---|---|---|---|
| Wirkstoff aus den Tabellen 1 und 2 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1 und 2 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1 und 2 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

| 2.8 Extruder Granulat | |
|---|---|
| Wirkstoff aus den Tabellen 1 und 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen 1 und 2 | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus den Tabellen 1 und 2 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

3.1 Wirkung gegen Meloidogyne incognita

Eier von Meloidogyne incognita werden in Sand eingemischt. Dieses Gemisch wird dann in 200 ml fassende Tontöpfe eingebracht (5000 Eier pro Topf). Am gleichen Tag wird pro Topf eine 3 Wochen alte Tomatenpflanze gepflanzt und der formulierte Wirkstoff mittels Druckapplikation in die Töpfe hineingegeben (0,002 % Aktivsubstanz bezogen auf das Bodenvolumen). Die eingetopften Pflanzen werden nun bei einer Temperatur von 26±1 °C und einer relativen Luftfeuchtigkeit von 60 % in einem Gewächshaus aufgestellt. Nach Ablauf von 4 Wochen erfolgt eine Evaluierung durch Untersuchung der Pflanzen auf Wurzelgallbildung nach dem sogenannten Wurzelgall-Index ("Knot Index").

Verbindungen aus den Tabellen 1 und 2 zeigen gegen Meloidogyne incognita durch weitgehende Reduktion der Wurzelgallbildung gute Wirksamkeit. Unbehandelte jedoch infizierte Kontrollpflanzen weisen dagegen starke Wurzelgallbildung auf (= 100 %). So hemmen z.B. die Verbindungen Nr. 1.1, 1.3 und 1.28 im obigen Versuch die Wurzelgallbildung fast vollständig (0-10 % Restbefall).

**Patentansprüche**

**Patentansprüche für folgende Vertragsstaaten : BE, CH, DE, FR, GB, IT, LI, LU, NL**

**1.** Verbindungen der Formel Ia

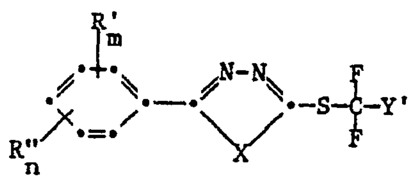

(Ia),

in welcher
R' $C_1$-$C_5$-Alkyl, $C_1$-$C_5$-Alkoxy, Halogen oder Trifluormethyl bedeutet,
R" $C_1$-$C_5$-Alkyl oder Halogen darstellt,
m für 1 bis 3 und n für 0 oder 1 stehen,
X Sauerstoff oder Schwefel bedeutet und
Y' Wasserstoff oder $CHF_2$ darstellt,
einschliesslich ihrer Säureadditionssalze.

**2.** Verbindungen der Formel Ib

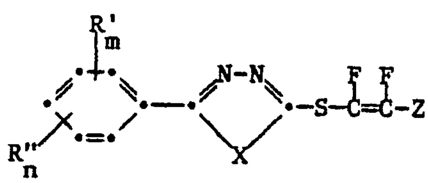

(Ib),

in welcher
R' $C_1$-$C_5$-Alkyl, $C_1$-$C_5$-Alkoxy, Halogen oder Trifluormethyl bedeutet,
R" $C_1$-$C_5$-Alkyl oder Halogen darstellt,
m für 1 bis 3 und n für 0 oder 1 stehen,
X Sauerstoff oder Schwefel bedeutet und
Z für Fluor oder Trifluormethyl steht, einschliesslich ihrer Säureadditionssalze.

**3.** Verbindungen der Formel I*

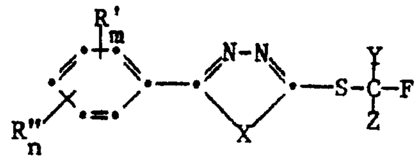 (I*),

in welcher

R' $C_1$-$C_5$-Alkyl, $C_1$-$C_5$-Alkoxy, Halogen oder Trifluormethyl bedeutet.
R" $C_1$-$C_5$-Alkyl oder Halogen darstellt,
m für 1 bis 3 und n für 0 oder 1 stehen,
X Sauerstoff oder Schwefel bedeutet,
Y Wasserstoff, Fluor Trifluormethyl oder die Gruppe HC(F)Z darstellt und
Z für Fluor oder Trifluormethyl steht, einschliesslich ihrer Säureadditionssalze.

**4.** Verbindungen der Formel Ia gemäss Anspruch 1, in denen R' $C_1$-$C_2$-Alkyl, $C_1$-$C_2$-Alkoxy, Halogen oder Trifluormethyl und R" $C_1$-$C_4$-Alkyl oder Halogen bedeuten, m für 1 oder 2 steht und X, Y', und n die unter Formel Ia angegebenen Bedeutungen besitzt.

**5.** Verbindungen der Formel Ia gemäss Anspruch 1, in denen R' Methyl, Methoxy, Fluor oder Chlor und R" Fluor oder Chlor bedeuten, Y' Wasserstoff darstellt und X und n die unter Formel Ia angegebenen Bedeutungen besitzt.

**6.** Verbindungen der Formel Ib gemäss Anspruch 2, in denen R' $C_1$-$C_2$-Alkyl, $C_1$-$C_2$-Alkoxy, Halogen oder Trifluormethyl und R" $C_1$-$C_4$-Alkyl oder Halogen bedeuten, m für 1 oder 2 steht und X, Z und n die unter Formel Ib angegebenen Bedeutungen besitzt.

**7.** Verbindungen der Formel Ib gemäss Anspruch 2, in denen R' Methyl, Methoxy, Fluor oder Chlor und R" Fluor oder Chlor bedeuten, Z Fluor darstellt und X und n die unter Formel Ib angegebenen Bedeutungen besitzt.

**8.** Verbindungen der Formel I* gemäss Anspruch 3, in denen R' $C_1$-$C_2$-Alkyl, $C_1$-$C_2$-Alkoxy, Halogen oder Trifluormethyl und R" $C_1$-$C_4$-Alkyl oder Halogen bedeuten, m für 1 oder 2 steht und X, Y, Z und n die unter Formel I* angegebenen Bedeutungen besitzt.

**9.** Verbindung der Formel I* gemäss Anspruch 3, in denen R' Methyl, Methoxy, Fluor oder Chlor und R" Fluor oder Chlor bedeuten, Y Wasserstoff und Z Fluor darstellen und Z und n die unter Formel I* angegebenen Bedeutungen besitzt.

**10.** Eine Verbindung ausgewählt aus der Gruppe:
2-Difluormethylthio-5-(4'-methoxyphenyl)-1,3,4-oxadiazol;
2-Difluormethylthio-5-(4'-methoxyphenyl)-1,3,4,-thiadiazol;
2-Difluormethylthio-5-(2'-fluorphenyl)-1,3,4-thiadiazol;
2-Difluormethylthio-5-(4'-chlorphenyl)-1,3,4-thiadiazol;
2-Difluormethylthio-5-(2'-methylphenyl)-1,3,4-thiadiazol;
2-Difluormethylthio-5-(2',4'-dichlorphenyl)-1,3,4,-thiadiazol;
2-Difluormethylthio-5-(2',6'-difluorphenyl)-1,3,4-thiadiazol;
2-Difluormethylthio-5-(4'-fluorphenyl)-1,3,4-thiadiazol;
2-Difluormethylthio-5-(3'-chlorphenyl)-1,3,4-thiadiazol

**11.** Verfahren zur Herstellung von Verbindungen der Formel Ia, Ib oder I* gemäss der Ansprüche 1-3, dadurch gekennzeichnet, dass man

a) in einer Kondensationsreaktion eine Verbindung der Formel IIa

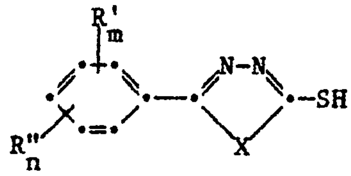

(IIa),

oder eine Verbindung der Formel IIb

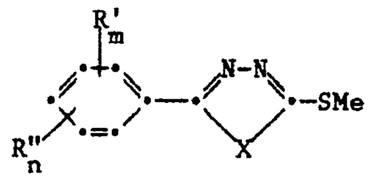

(IIb),

mit einer Verbindung der Formel IIIa

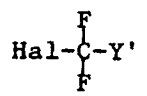

(IIIa)

oder mit einer Verbindung der Formel III*

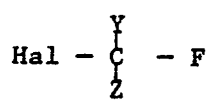

(III*)

in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel IIa in Gegenwart einer Base verläuft, oder

b) in einer Anlagerungsreaktion eine Verbindung der Formel IIa

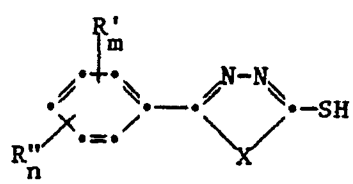

(IIa),

mit einer Verbindung der Formel IIIb

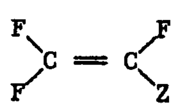

(IIIb)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ia*

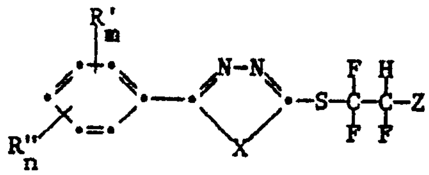

(Ia*),

oder zu einer Verbindung der Formel Ib

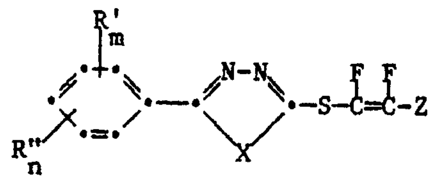

(Ib),

führt, wobei in den vorstehend genannten Formeln IIa, IIb, Ia*, Ib, IIIa, IIIb und III* Me ein Alkalimetall oder Ammonium, Hal Halogen, vorzugsweise Chlor, Brom oder Jod bedeuten, während R', R", m, n, Y, Y' und Z die unter den Formeln Ia, Ib oder I* angegebenen Bedeutungen besitzen.

**12.** Schädlingsbekämpfungsmittel zur Bekämpfung oder Verhütung des Befalls von Pflanzen durch Nematoden, dadurch gekennzeichnet, dass es als aktive Komponente mindestens eine Verbindung der Formeln Ia, Ib oder I* gemäss den Ansprüchen 1 bis 3 enthält.

**13.** Mittel gemäss Anspruch 12, dadurch gekennzeichnet, dass es als aktive Komponente mindestens eine Verbindung der Formeln Ia, Ib oder I* gemäss den Ansprüchen 4 bis 10 enthält.

**14.** Mittel nach einem der Ansprüche 12 und 13, dadurch gekennzeichnet, dass es 0,1 bis 99 % eines Wirkstoffs der Formel Ia, Ib oder I* 99,9 bis 1 % eines festen oder flüssigen Zusatzstoffes und 0 bis 25 % eines Tensides enthält.

**15.** Mittel nach Anspruch 14, dadurch gekennzeichnet, dass es 0,1 bis 95 % eines Wirkstoffes der Formel Ia, Ib oder I*, 99,8 bis 5 % eines festen oder flüssigen Zusatzstoffes und 0,1 bis 25 % eines Tensides enthält.

**16.** Verfahren zur Herstellung eines agrochemischen Mittels, dadurch gekennzeichnet, dass mindestens eine Verbindung der Formeln Ia, Ib oder I* gemäss einem der Ansprüche 12 bis 15 mit geeigneten festen oder flüssigen Zusatzstoffen und Tensiden innig vermischt wird.

**17.** Verfahren zur Bekämpfung oder Verhütung eines Befalls von Kulturpflanzen durch Nematoden, dadurch gekennzeichnet, dass man eine Verbindung der Formeln Ia, Ib oder I* gemäss den Ansprüchen 1 bis 3 auf die Pflanze oder deren Standort appliziert.

**18.** Verwendung von Verbindungen der Formeln Ia, Ib oder I* gemäss den Ansprüchen 1 bis 3 zur Bekämpfung und/oder zur präventiven Verhütung eines Befalls von Pflanzen durch Nematoden.

**19.** Verwendung gemäss Anspruch 18, von Verbindungen der Formeln Ia, Ib oder I* gemäss den Ansprüchen 4 bis 10.

**20.** Verwendung gemäss Anspruch 18, dadurch gekennzeichnet, dass es sich bei den Nematoden um Pflanzenwurzel-parasitäre Species handelt.

**21.** Verwendung gemäss Anspruch 20 gegen Nematoden der Gattung Meloidogyne, Heterodera oder Globodera.

**Patentansprüche für folgenden Vertragsstaat : AT**

**1.** Schädlingsbekämpfungsmittel zur Bekämpfung oder Verhütung des Befalls von Pflanzen durch Nematoden enthaltend neben üblichen Träger- und Hilfsstoffen als aktive Komponente mindestens eine Verbindung der Formel Ia

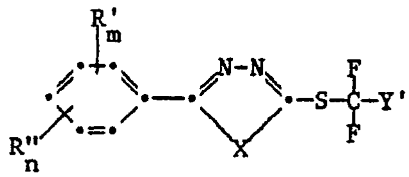

(Ia),

in welcher
- R' $C_1$-$C_5$-Alkyl, $C_1$-$C_5$-Alkoxy, Halogen oder Trifluormethyl bedeutet,
- R" $C_1$-$C_5$-Alkyl oder Halogen darstellt,
- m für 1 bis 3 und n für 0 oder 1 stehen,
- X Sauerstoff oder Schwefel bedeutet und
- Y' Wasserstoff oder $CHF_2$ darstellt,

einschliesslich ihrer Säureadditionssalze.

**2.** Schädlingsbekämpfungsmittel zur Bekämpfung oder Verhütung des Befalls von Pflanzen durch Nematoden enthaltend neben üblichen Träger- und Hilfsstoffen als aktive Komponente mindestens eine Verbindung der Formel Ib

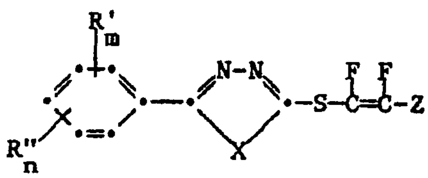

(Ib),

in welcher
- R' $C_1$-$C_5$-Alkyl, $C_1$-$C_5$-Alkoxy, Halogen oder Trifluormethyl bedeutet,
- R" $C_1$-$C_5$-Alkyl oder Halogen darstellt,
- m für 1 bis 3 und n für 0 oder 1 stehen,
- X Sauerstoff oder Schwefel bedeutet und
- Z für Fluor oder Trifluormethyl steht, einschliesslich ihrer Säureadditionssalze.

**3.** Schädlingsbekämpfungsmittel zur Bekämpfung oder Verhütung des Befalls von Pflanzen durch Nematoden enthaltend neben üblichen Träger- und Hilfsstoffen als aktive Komponente mindestens eine Verbindung der Formel I*

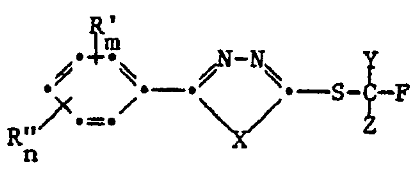

(I*),

in welcher
- R' $C_1$-$C_5$-Alkyl, $C_1$-$C_5$-Alkoxy, Halogen oder Trifluormethyl bedeutet.
- R" $C_1$-$C_5$-Alkyl oder Halogen darstellt,
- m für 1 bis 3 und n für 0 oder 1 stehen,
- X Sauerstoff oder Schwefel bedeutet,
- Y Wasserstoff, Fluor, Trifluormethyl oder die Gruppe HC(F)Z darstellt und
- Z für Fluor oder Trifluormethyl steht, einschliesslich ihrer Säureadditionssalze.

**4.** Mittel nach Anspruch 1 enthaltend als Aktivkomponente mindestens eine Verbindung der Formel Ia, worin R' $C_1$-$C_2$-Alkyl, $C_1$-$C_2$-Alkoxy, Halogen oder Trifluormethyl und R" $C_1$-$C_4$-Alkyl oder Halogen

bedeuten, m für 1 oder 2 steht und X, Y', und n die unter Formel Ia angegebenen Bedeutungen besitzt.

5. Mittel nach Anspruch 1 enthaltend als Aktivkomponente mindestens eine Verbindung der Formel Ia, worin R' Methyl, Methoxy, Fluor oder Chlor und R" Fluor oder Chlor bedeuten, Y' Wasserstoff darstellt und X und n die unter Formel Ia angegebenen Bedeutungen besitzt.

6. Mittel nach Anspruch 2 enthaltend als Aktivkomponente mindestens eine Verbindung der Formel Ib, worin R' $C_1$-$C_2$-Alkyl, $C_1$-$C_2$-Alkoxy, Halogen oder Trifluormethyl und R" $C_1$-$C_4$-Alkyl oder Halogen bedeuten, m für 1 oder 2 steht und X, Z und n die unter Formel Ib angegebenen Bedeutungen besitzt.

7. Mittel nach Anspruch 2 enhaltend als Aktivkomponente mindestens eine Verbindung der Formel Ib, worin R' Methyl, Methoxy, Fluor oder Chlor und R" Fluor oder Chlor bedeuten, Z Fluor darstellt und X und n die unter Formel Ib angegebenen Bedeutungen besitzt.

8. Mittel nach Anspruch 3 enthaltend als Aktivkomponente mindestens eine Verbindung der Formel I* worin R' $C_1$-$C_2$-Alkyl, $C_1$-$C_2$-Alkoxy, Halogen oder Trifluormethyl und R" $C_1$-$C_4$-Alkyl oder Halogen bedeuten, m für 1 oder 2 steht und X, Y, Z und n die unter Formel I* angegebenen Bedeutungen besitzt.

9. Mittel nach Anspruch 3 enthaltend als Aktivkomponente mindestens eine Verbindung der Formel I*, worin R' Methyl, Methoxy, Fluor oder Chlor und R" Fluor oder Chlor bedeuten, Y Wasserstoff und Z Fluor darstellen und X und n die unter Formel I* angegebenen Bedeutungen besitzt.

10. Mittel nach Anspruch 1 enthaltend als Aktivkomponente mindestens eine Verbindung der Formel Ia ausgewählt aus der Gruppe:
2-Difluormethylthio-5-(4'-methoxyphenyl)-1,3,4-oxadiazol;
2-Difluormethylthio-5-(4'-methoxyphenyl)-1,3,4,-thiadiazol;
2-Difluormethylthio-5-(2'-fluorphenyl)-1,3,4-thiadiazol;
2-Difluormethylthio-5-(4'-chlorphenyl)-1,3,4-thiadiazol;
2-Difluormethylthio-5-(2'-methylphenyl)-1,3,4-thiadiazol;
2-Difluormethylthio-5-(2',4'-dichlorphenyl)-1,3,4-thiadiazol;
2-Difluormethylthio-5-(2',6'-difluorphenyl)-1,3,4-thiadiazol;
2-Difluormethylthio-5-(4'-fluorphenyl)-1,3,4-thiadiazol;
2-Difluormethylthio-5-(3'-chlorphenyl)-1,3,4-thiadiazol.

11. Mittel nach einem der Ansprüche 1 bis 10, dadurch gekennzeichnet, dass es 0,1 bis 99 % eines Wirkstoffs der Formel Ia, Ib oder I*, 99,9 bis 1 % eines festen oder flüssigen Zusatzstoffes und 0 bis 25 % eines Tensides enthält.

12. Mittel nach Anspruch 11, dadurch gekennzeichnet, dass es 0,1 bis 95 % eines Wirkstoffes der Formel Ia, Ib oder I*, 99,8 bis 5 % eines festen oder flüssigen Zusatzstoffes und 0,1 bis 25 % eines Tensides enthält.

13. Verfahren zur Herstellung von Verbindungen der Formeln Ia, Ib und I* aus den Ansprüchen 1-3, dadurch gekennzeichnet, dass man

a) in einer Kondensationsreaktion eine Verbindung der Formel IIa

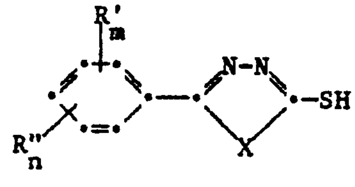

(IIa),

oder eine Verbindung der Formel IIb

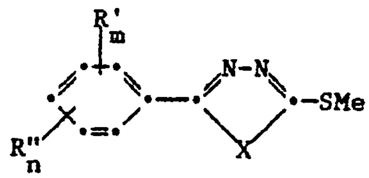

(IIb),

mit einer Verbindung der Formel IIIa

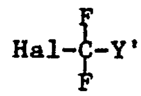

(IIIa)

oder mit einer Verbindung der Formel III*

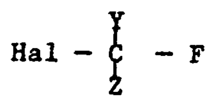

(III*)

in einem inerten Lösungsmittel oder Lösungsmittel-Gemisch bei erhöhter Temperatur gegebenenfalls in Gegenwart eines Katalysators und gegebenenfalls bei erhöhtem Druck umsetzt, wobei die Umsetzung einer Verbindung der Formel IIa in Gegenwart einer Base verläuft, oder

b) in einer Anlagerungsreaktion eine Verbindung der Formel IIa

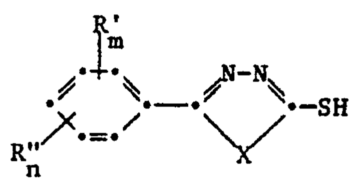

(IIa),

mit einer Verbindung der Formel IIIb

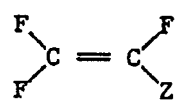

(IIIb)

in einem inerten Lösungsmittel oder Lösungsmittel-Gemisch bei erhöhter Temperatur gegebenenfalls in Gegenwart eines Katalysators und gegebenenfalls bei erhöhtem Druck reagieren lässt, wobei diese Reaktion zu einer Verbindung der Formel Ia*

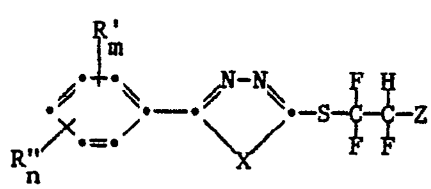

(Ia*),

oder zu einer Verbindung der Formel Ib

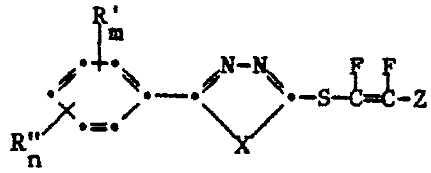

(Ib),

führt, wobei in den vorstehend genannten Formeln IIa, IIb, Ia*, Ib, IIIa, IIIb und III* Me ein Alkalimetall oder Ammonium, Hal Halogen, vorzugsweise Chlor, Brom oder Jod bedeuten, während R', R'', m, n, Y, Y' und Z die unter den Formeln Ia, Ib oder I* angegebenen Bedeutungen besitzen.

**14.** Verfahren zur Herstellung eines agrochemischen Mittels, dadurch gekennzeichnet, dass mindestens eine Verbindung der Formeln Ia, Ib oder I* gemäss einem der Ansprüche 1 bis 3 mit geeigneten festen oder flüssigen Zusatzstoffen und Tensiden innig vermischt wird.

**15.** Verfahren zur Bekämpfung oder Verhütung eines Befalls von Kulturpflanzen durch Nematoden, dadurch gekennzeichnet, dass man eine Verbindung der Formeln Ia, Ib oder I* gemäss den Ansprüchen 1 bis auf die Pflanze oder deren Standort appliziert.

**16.** Verwendung von Verbindungen der Formeln Ia, Ib oder I* gemäss den Ansprüchen 1 bis 3 zur Bekämpfung und/oder zur präventiven Verhütung eines Befalls von Pflanzen durch Nematoden.

**17.** Verwendung gemäss Anspruch 16, dadurch gekennzeichnet, dass es sich bei den Nematoden um Pflanzenwurzel-parasitäre Species handelt.

**18.** Verwendung gemäss Anspruch 17 gegen Nematoden der Gattung Meloidogyne, Heterodera oder Globodera.

**Claims**

**Claims for the following Contracting States : BE, CH, DE, FR, GB, IT, LI, LU, NL,**

**1.** A compound of formula Ia

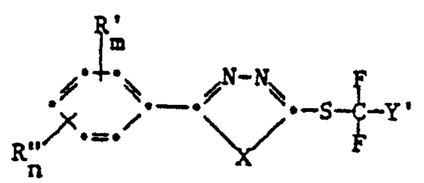

(Ia).

in which
R' is $C_1$-$C_5$alkyl, $C_1$-$C_5$alkoxy, halogen or trifluoromethyl,
R'' is $C_1$-$C_5$alkyl or halogen,
m is 1 to 3 and n is 0 or 1,
X is oxygen or sulfur and
Y' is hydrogen or $CHF_2$,
or an acid addition salt thereof.

**2.** A compound of formula Ib

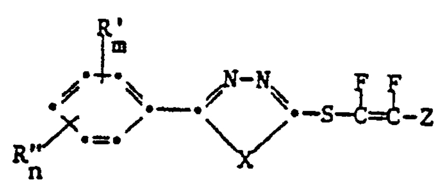

(Ib).

in which
R' is $C_1$-$C_5$alkyl, $C_1$-$C_5$alkoxy, halogen or trifluoromethyl,
R'' is $C_1$-$C_5$alkyl or halogen,
m is 1 to 3 and n is 0 or 1,
X is oxygen or sulfur and
Z is fluorine or trifluoromethyl,
or an acid addition salt thereof.

3. A compound of formula I*

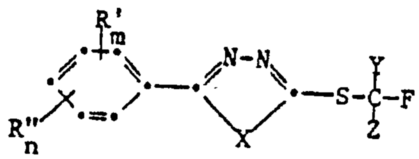

(I*).

in which
R' is $C_1$-$C_5$alkyl, $C_1$-$C_5$alkoxy, halogen or trifluoromethyl,
R'' is $C_1$-$C_5$alkyl or halogen,
m is 1 to 3 and n is 0 or 1,
X is oxygen or sulfur,
Y is hydrogen, fluorine, trifluoromethyl or the group HC(F)Z and
Z is fluorine or trifluoromethyl,
or an acid addition salt thereof.

4. A compound of formula Ia according to claim 1, in which R' is $C_1$-$C_2$alkyl, $C_1$-$C_2$alkoxy, halogen or trifluoromethyl and R'' is $C_1$-$C_4$alkyl or halogen, m is 1 or 2 and X, Y' and n are as defined for formula Ia.

5. A compound of formula Ia according to claim 1, in which R' is methyl, methoxy, fluorine or chlorine and R'' is fluorine or chlorine, Y' is hydrogen and X and n are as defined for formula Ia.

6. A compound of formula Ib according to claim 2, in which R' is $C_1$-$C_2$alkyl, $C_1$-$C_2$alkoxy, halogen or trifluoromethyl and R'' is $C_1$-$C_4$alkyl or halogen, m is 1 or 2 and X, Z and n are as defined for formula Ib.

7. A compound of formula Ib according to claim 2, in which R' is methyl, methoxy, fluorine or chlorine and R'' is fluorine or chlorine, Z is fluorine and X and n are as defined for formula Ib.

8. A compound of formula I* according to claim 3, in which R' is $C_1$-$C_2$alkyl, $C_1$-$C_2$alkoxy, halogen or trifluoromethyl and R'' is $C_1$-$C_4$alkyl or halogen, m is 1 or 2 and X, Y, Z and n are as defined for formula I*.

9. A compound of formula I* according to claim 3, in which R' is methyl, methoxy, fluorine or chlorine and R'' is fluorine or chlorine, Y is hydrogen and Z is fluorine and X and n are as defined for formula I*.

10. A compound selected from the group consisting of
2-difluoromethylthio-5-(4'-methoxyphenyl)-1,3,4-oxadiazole;
2-difluoromethylthio-5-(4'-methoxyphenyl)-1,3,4-thiadiazole;
2-difluoromethylthio-5-(2'-fluorophenyl)-1,3,4-thiadiazole;
2-difluoromethylthio-5-(4'-chlorophenyl)-1,3,4-thiadiazole;
2-difluoromethylthio-5-(2'-methylphenyl)-1,3,4-thiadiazole;
2-difluoromethylthio-5-(2',4'-dichlorophenyl)-1,3,4-thiadiazole;
2-difluoromethylthio-5-(2',6'-difluorophenyl)-1,3,4-thiadiazole;
2-difluoromethylthio-5-(4'-fluorophenyl)-1,3,4-thiadiazole;
2-difluoromethylthio-5-(3'-chlorophenyl)-1,3,4-thiadiazole.

**11.** A process for the preparation of a compound of formula Ia, Ib or I* as defined in any one of claims 1 to 3, which comprises

a) reacting, in a condensation reaction, a compound of formula IIa

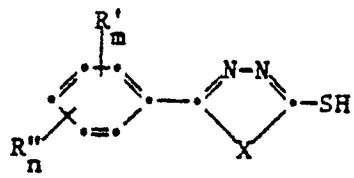

(IIa),

or a compound of formula IIb

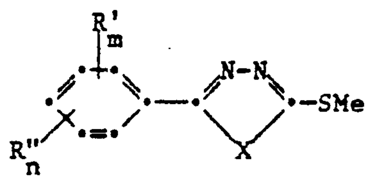

(IIb)

with a compound of formula IIIa

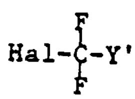

(IIIa)

or with a compound of formula III*

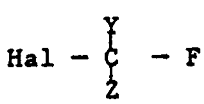

(III*)

in an inert solvent or solvent mixture, at elevated temperature, in the absence or presence of a catalyst and, if desired, under increased pressure, the reaction of a compound of formula IIa being carried out in the presence of a base, or

b) reacting, in an addition reaction, a compound of formula IIa

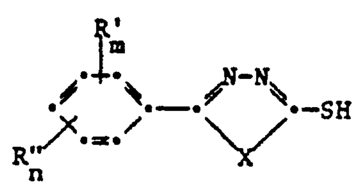

(IIa),

with a compound of formula IIIb

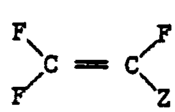

(IIIb)

in an inert solvent or solvent mixture, at elevated temperature, in the absence or presence of a catalyst and, if desired, under increased pressure, this reaction affording a compound of formula Ia*

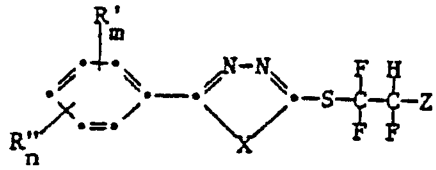

(Ia*),

or a compound of formula Ib

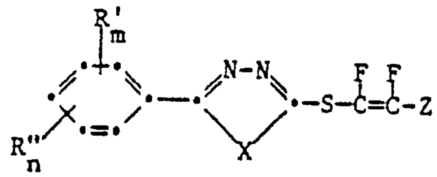

(Ib),

where in the above formulae IIa, IIb, Ia*, Ib, IIIa, IIIb and III*, Me is an alkali metal or ammonium, Hal is halogen, preferably chlorine, bromine or iodine, and R', R'', m, n, Y, Y' and Z are as defined for formulae Ia, Ib and I*.

**12.** A pesticidal composition for controlling nematodes or for protecting plants from attack by nematodes, which contains as active ingredient at least one compound of formula Ia, Ib or I* as defined in any one of claims 1 to 3.

**13.** A composition according to claim 12, which contains as active ingredient at least one compound of formula Ia, Ib or I* as defined in any one of claims 4 to 10.

**14.** A composition according to either of claims 12 or 13, which contains 0.1 to 99 % of an active substance of formula Ia, Ib or I*, 99.9 to 1 % of a solid or liquid adjuvant and 0 to 25 % of a surfactant.

**15.** A composition according to claim 14, which contains 0.1 to 95 % of an active substance of formula Ia, Ib or I*, 99.8 to 5 % of a solid or liquid adjuvant and 0.1 to 25 % of a surfactant.

**16.** A process for the preparation of an agrochemical composition, which comprises homogeneously mixing at least one compound of formula Ia, Ib or I* as defined in any of claims 12 to 15 with suitable solid or liquid adjuvants and surfactants.

**17.** A method of controlling nematodes or of protecting cultivated plants from attack by nematodes, which comprises applying to the plant or to the locus thereof a compound of formula Ia, Ib or I* as defined in any one of claims 1 to 3.

**18.** The use of a compound of formula Ia, Ib or I* as defined in any one of claims 1 to 3 for controlling nematodes and/or preventively protecting plants from attack by nematodes.

**19.** The use according to claim 18 of a compound of formula Ia, Ib or I* as defined in any one of claims 4 to 10.

**20.** The use according to claim 18, wherein the nematodes are species which parasitise in the roots of plants.

**21.** The use according to claim 20, against nematodes of the genus Meloidogyne, Heterodera or Globodera.

**Claims for the following Contracting State : AT**

**1.** A pesticidal composition for controlling nematodes or protecting plants from attack by nematodes, which contains in addition to conventional carriers and assistants, as active ingredient, at least one compound of formula Ia

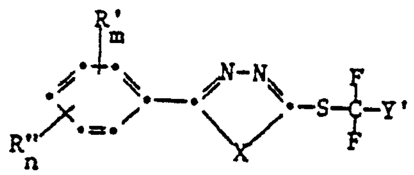

(Ia),

in which
R' is $C_1$-$C_5$alkyl, $C_1$-$C_5$alkoxy, halogen or trifluoromethyl,
R" is $C_1$-$C_5$alkyl or halogen,
m is 1 to 3 and n is 0 or 1,
X is oxygen or sulfur and
Y' is hydrogen or $CHF_2$,
or an acid addition salt thereof.

2. A pesticidal composition for controlling nematodes or protecting plants from attack by nematodes, which contains in addition to conventional carriers and assistants, as active ingredient, at least one compound of formula Ib

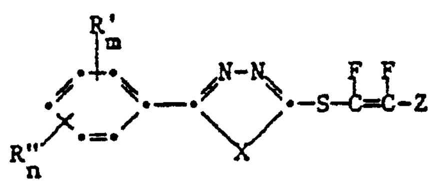

(Ib),

in which
R' is $C_1$-$C_5$alkyl, $C_1$-$C_5$alkoxy, halogen or trifluoromethyl,
R" is $C_1$-$C_5$alkyl or halogen,
m is 1 to 3 and n is 0 or 1,
X is oxygen or sulfur and
Z is fluorine or trifluoromethyl,
or an acid addition salt thereof.

3. A pesticidal composition for controlling nematodes or protecting plants from attack by nematodes, which contains in addition to conventional carriers and assistants, as active ingredient, at least one compound of formula I*

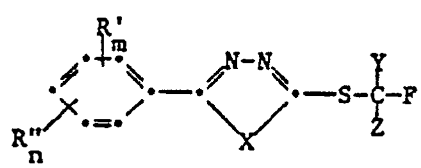

(I*),

in which
R' is $C_1$-$C_5$alkyl, $C_1$-$C_5$alkoxy, halogen or trifluoromethyl,
R" is $C_1$-$C_5$alkyl or halogen,
m is 1 to 3 and n is 0 or 1,
X is oxygen or sulfur and
Y is hydrogen, fluorine, trifluoromethyl or the group HC(F)Z and
Z is fluorine or trifluoromethyl,
or an acid addition salt thereof.

4. A composition according to claim 1, which contains as active ingredient at least one compound of formula Ia, in which R' is $C_1$-$C_2$alkyl, $C_1$-$C_2$alkoxy, halogen or trifluoromethyl and R" is $C_1$-$C_4$alkyl or halogen, m is 1 or 2 and X, Y' and n are as defined for formula Ia.

5. A composition according to claim 1, which contains as active ingredient at least one compound of formula Ia, in which R' is methyl, methoxy, fluorine or chlorine and R'' is fluorine or chlorine, Y' is hydrogen and X and n are as defined for formula Ia.

6. A composition according to claim 2, which contains as active ingredient at least one compound of formula Ib, in which R' is $C_1$-$C_2$alkyl, $C_1$-$C_2$alkoxy, halogen or trifluoromethyl and R'' is $C_1$-$C_4$alkyl or halogen, m is 1 or 2 and X, Z and n are as defined for formula Ib.

7. A composition according to claim 2, which contains as active ingredient at least one compound of formula Ib, in which R' is methyl, methoxy, fluorine or chlorine and R'' is fluorine or chlorine, Z is fluorine and X and n are as defined for formula Ib.

8. A composition according to claim 3, which contains as active ingredient at least one compound of formula I*, in which R' is $C_1$-$C_2$alkyl, $C_1$-$C_2$alkoxy, halogen or trifluoromethyl and R'' is $C_1$-$C_4$alkyl or halogen, m is 1 or 2 and X, Y, Z and n are as defined for formula I*.

9. A composition according to claim 3, which contains as active ingredient at least one compound of formula I*, in which R' is methyl, methoxy, fluorine or chlorine and R'' is fluorine or chlorine, Y is hydrogen and Z is fluorine and X and n are as defined for formula I*.

10. A composition according to claim 1, which contains as active ingredient at least one compound of formula Ia selected from the group consisting of
2-difluoromethylthio-5-(4'-methoxyphenyl)-1,3,4-oxadiazole;
2-difluoromethylthio-5-(4'-methoxyphenyl)-1,3,4-thiadiazole;
2-difluoromethylthio-5-(2'-fluorophenyl)-1,3,4-thiadiazole;
2-difluoromethylthio-5-(4'-chlorophenyl)-1,3,4-thiadiazole;
2-difluoromethylthio-5-(2'-methylphenyl)-1,3,4-thiadiazole;
2-difluoromethylthio-5-(2',4'-dichlorophenyl)-1,3,4-thiadiazole;
2-difluoromethylthio-5-(2',6'-difluorophenyl)-1,3,4-thiadiazole;
2-difluoromethylthio-5-(4'-fluorophenyl)-1,3,4-thiadiazole;
2-difluoromethylthio-5-(3'-chlorophenyl)-1,3,4-thiadiazole.

11. A composition according to any one of claims 1 to 10, which contains 0.1 to 99 % of an active substance of formula Ia, Ib or I*, 99.9 to 1 % of a solid or liquid adjuvant and 0 to 25 % of a surfactant.

12. A composition according to claim 11, which contains 0.1 to 95 % of an active substance of formula Ia, Ib or I*, 99.8 to 5 % of a solid or liquid adjuvant and 0.1 to 25 % of a surfactant.

13. A process for the preparation of a compound of formula Ia, Ib or I* as defined in any one of claims 1 to 3, which comprises

a) reacting, in a condensation reaction, a compound of formula IIa

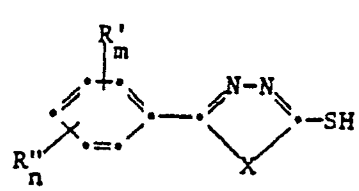

(IIa)

or a compound of formula IIb

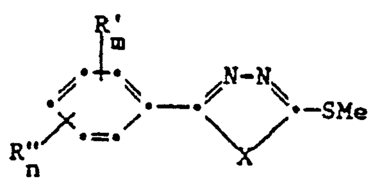

(IIb)

with a compound of formula IIIa

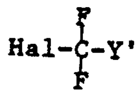

(IIIa)

or with a compound of formula III*

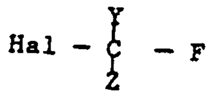

(III*)

in an inert solvent or solvent mixture, at elevated temperature in the absence or presence of a catalyst and, if desired, under increased pressure, the reaction of a compound of formula IIa being carried out in the presence of a base, or

b) reacting, in an addition reaction, a compound of formula IIa

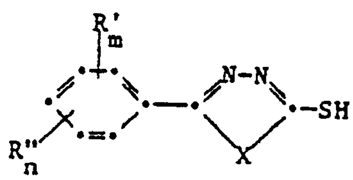

(IIa),

with a compound of formula IIIb

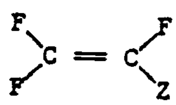

(IIIb)

in an inert solvent or solvent mixture, at elevated temperature, in the absence or presence of a catalyst and, if desired, under increased pressure, this reaction affording a compound of formula Ia*

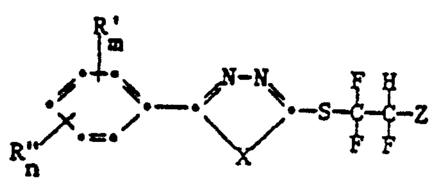

(Ia*).

or a compound of formula Ib

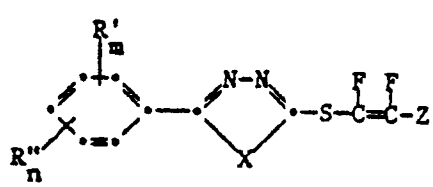

(Ib),

where in the above formulae IIa, IIb, Ia*, Ib, IIIa, IIIb and III*, Me is an alkali metal or ammonium, Hal is halogen, preferably chlorine, bromine or iodine, and R', R'', m, n, Y, Y' and Z are as defined for formulae Ia, Ib and I*.

**14.** A process for the preparation of an agrochemical composition, which comprises homogeneously mixing at least one compound of formula Ia, Ib or I* as defined in any one of claims 1 to 3 with suitable solid or liquid adjuvants and surfactants.

**15.** A method of controlling nematodes or of protecting cultivated plants from attack by nematodes, which comprises applying to the plant or to the locus thereof a compound of formula Ia, Ib or I* as defined in any one of claims 1 to 3.

**16.** The use of a compound of formula Ia, Ib or I* as defined in any one of claims 1 to 3 for controlling nematodes and/or preventively protecting plants from attack by nematodes.

**17.** The use according to claim 16, wherein the nematodes are species which parasitise in the roots of plants.

**18.** The use according to claim 17, against nematodes of the genus Meloidogyne, Heterodera or Globodera.

**Revendications**
**Revendications pour les Etats contractants suivants : BE, CH, DE, FR, GB, IT, LI, LU, NL**

**1.** Composés de formule Ia

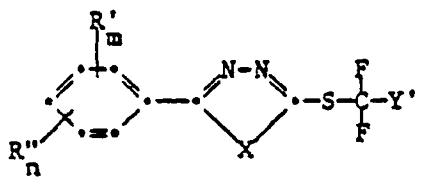
(Ia),

dans laquelle

R' représente un alkyle en $C_1$-$C_5$, un alcoxy en $C_1$-$C_5$, un halogène ou le trifluorométhyle,
R" représente un alkyle en $C_1$-$C_5$ ou un halogène,
m est égal à 1, 2 ou 3 et n est égal à 0 ou 1,
X représente l'oxygène ou le soufre et
Y' représente l'hydrogène ou $CHF_2$,

y compris leurs sels d'addition d'acides.

**2.** Composés de formule Ib

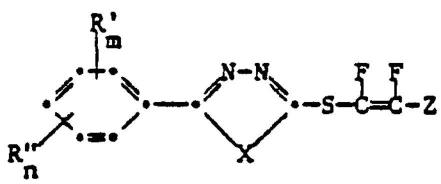
(Ib),

dans laquelle

R' représente un alkyle en $C_1$-$C_5$, un alcoxy en $C_1$-$C_5$, un halogène ou le trifluorométhyle,
R" représente un alkyle en $C_1$-$C_5$ ou un halogène,
m est égal à 1, 2 ou 3 et n est égal à 0 ou 1,
X représente l'oxygène ou le soufre et
Z représente le fluor ou le trifluorométhyle,

y compris leurs sels d'addition d'acides.

**3.** Composés de formule I*

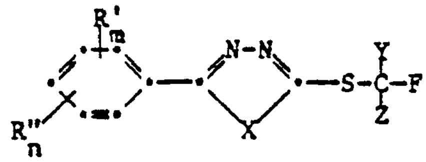

(I*),

dans laquelle

R' représente un alkyle en $C_1$-$C_5$, un alcoxy en $C_1$-$C_5$, un halogène ou le trifluorométhyle,
R" représente un alkyle en $C_1$-$C_5$ ou un halogène,
m est égal à 1, 2 ou 3 et n est égal à 0 ou 1,
X représente l'oxygène ou le soufre,
Y représente l'hydrogène, le fluor, le trifluorométhyle ou le groupe HC(F)Z et
Z représente le fluor ou le trifluorométhyle,

y compris leurs sels d'addition d'acides.

**4.** Composés de formule Ia selon la revendication 1, dans laquelle R' représente un alkyle en $C_1$-$C_2$, un alcoxy en $C_1$-$C_2$, un halogène ou le trifluorométhyle et R" représente un alkyle en $C_1$-$C_4$ ou un halogène, m est égal à 1 ou 2 et X, Y' et n ont les significations indiquées pour la formule Ia.

**5.** Composés de formule Ia selon la revendication 1, dans laquelle R' représente le méthyle, le méthoxy, le fluor ou le chlore et R" représente le fluor ou le chlore, Y' représente l'hydrogène et X et n ont les significations indiquées pour la formule Ia.

**6.** Composés de formule Ib selon la revendication 2, dans laquelle R' représente un alkyle en $C_1$-$C_2$, un alcoxy en $C_1$-$C_2$, un halogène ou le trifluorométhyle et R" représente un alkyle en $C_1$-$C_4$ ou un halogène, n est égal à 1 ou 2 et X, Z et n ont les significations indiquées pour la formule Ib.

**7.** Composés de formule Ib selon la revendication 2, dans laquelle R' représente le méthyle, le méthoxy, le fluor ou le chlore et R" représente le fluor ou le chlore, Z représente le fluor et X et n ont les significations indiquées pour la formule Ib.

**8.** Composés de formule I* selon la revendication 3, dans laquelle R' représente un alkyle en $C_1$-$C_2$, un alcoxy en $C_1$-$C_2$, un halogène ou le trifluorométhyle et R" représente un alkyle en $C_1$-$C_4$ ou un halogène, m est égal à 1 ou 2 et X, Y, Z et n ont les significations indiquées pour la formule I*.

**9.** Composés de formule I* selon la revendication 3, dans laquelle R' représente le méthyle, le méthoxy, le fluor ou le chlore et R" représente le fluor ou le chlore, Y représente l'hydrogène et Z représente le fluor et X et n ont les significations indiquées pour la formule I*.

**10.** Un composé choisi dans le groupe :
2-difluorométhylthio-5-(4'-méthoxyphényl)-1,3,4-oxadiazole;
2-difluorométhylthio-5-(4'-méthoxyphényl)-1,3,4-thiadiazole;
2-difluorométhylthio-5-(2'-fluorophényl)-1,3,4-thiadiazole;
2-difluorométhylthio-5-(4'-chlorophényl)-1,3,4-thiadiazole;
2-difluorométhylthio-5-(2'-méthylphényl)-1,3,4-thiadiazole;
2-difluorométhylthio-5-(2',4'-dichlorophényl)-1,3,4-thiadiazole;
2-difluorométhylthio-5-(2',6'-difluorophényl)-1,3,4-thiadiazole;
2-difluorométhylthio-5-(4'-fluorophényl)-1,3,4-thiadiazole;
2-difluorométhylthio-5-(3'-chlorophényl)-1,3,4-thiadiazole.

**11.** Procédé pour la préparation des composés de formules Ia, Ib ou I* selon les revendications 1-3, caractérisé

a) en ce que l'on fait réagir, dans une réaction de condensation, un composé de formule IIa

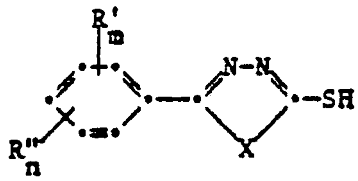

(IIa),

ou un composé de formule IIb

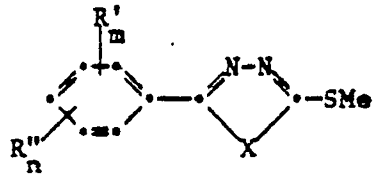

(IIb),

avec un composé de formule IIIa

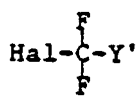

(IIIa)

ou avec un composé de formule III*

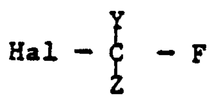

(III*)

dans un solvant ou dans un mélange de solvants inertes à une température augmentée, éventuellement en présence d'un catalyseur et éventuellement à une pression augmentée, la réaction d'un composé de formule IIa s'effectuant, en présence d'une base ou

b) en ce que l'on fait réagir, dans une réaction d'addition, un composé de formule IIa

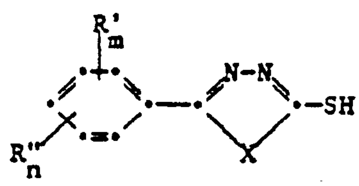

(IIa),

avec un composé de formule IIIb

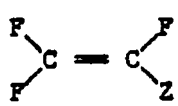

(IIIb)

dans un solvant ou dans un mélange de solvants inertes à une température augmentée, éventuellement en présence d'un catalyseur et éventuellement à une pression augmentée, cette réaction conduisant à un composé de formule Ia*

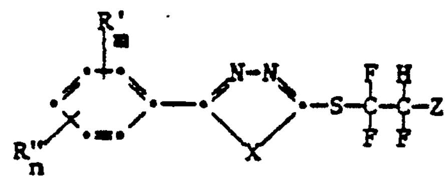

(Ia*),

ou à un composé de formule Ib

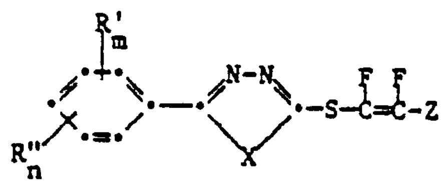

(Ib),

dans les formules IIa, IIb, Ia*, Ib, IIIa, IIIb et III* précédemment citées, Me désigne un métal alcalin ou l'ammonium, Hal un halogène, de préférence le chlore, le brome ou l'iode, tandis que R', R", m, n, Y, Y' et Z ont les significations indiquées pour les formules Ia, Ib ou I*.

12. Agent antiparasitaire pour lutter contre ou prévenir l'infestation des plantes par des nématodes, caractérisé en ce qu'il contient, comme composant actif, au moins un composé de formules Ia, Ib ou I* selon les revendications 1 à 3.

13. Agent selon la revendication 12, caractérisé en ce qu'il contient, comme composant actif, au moins un composé de formules Ia, Ib ou I* selon les revendications 4 à 10.

14. Agent selon l'une des revendications 12 et 13, caractérisé en ce qu'il contient 0,1 à 99 % d'une substance active de formules Ia, Ib ou I*, 99,9 à 1 % d'un additif solide ou liquide et 0 à 25 % d'un agent de surface.

15. Agent selon la revendication 14, caractérisé en ce qu'il contient 0,1 à 95 % d'une substance active de formules Ia, Ib ou I*, 99,8 à 5 % d'un additif solide ou liquide et 0,1 à 25 % d'un agent de surface.

16. Procédé pour la préparation d'un agent agrochimique, caractérisé en ce que l'on mélange intimement au moins un composé de formules Ia, Ib ou I* selon l'une des revendications 12 à 15, avec des additifs solides ou liquides appropriés et des agents de surface.

17. Procédé pour lutter contre ou prévenir une infestation des plantes cultivées par des nématodes, caractérisé en ce que l'on applique un composé de formules Ia, Ib ou I* selon les revendications 1 à 3 sur la plante ou sur son emplacement.

18. Utilisation des composés de formules Ia, Ib ou I* selon les revendications 1 à 3 pour lutter contre et/ou prévenir l'infestation des plantes par des nématodes.

19. Utilisation selon la revendication 18, des composés de formules Ia, Ib ou I* selon les revendications 4 à 10.

20. Utilisation selon la revendication 18, caractérisée en ce que les nématodes sont des espèces parasitant les racines des plantes.

21. Utilisation contre les nématodes du genre Meloidogyne, Heterodera ou Globodera, selon la revendication 20.

**Revendications pour l'Etat contractant suivant : AT**

1. Agents antiparasitaires pour lutter contre ou prévenir l'infestation des plantes par des nématodes contenant, à côté des supports et adjuvants usuels, comme composant actif, au moins un composé de formule Ia

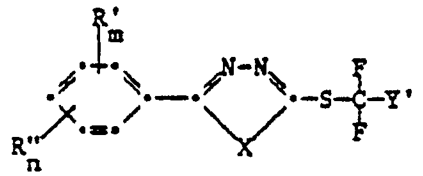

(Ia),

dans laquelle

R' représente un alkyle en $C_1$-$C_5$, un alcoxy en $C_1$-$C_5$, un halogène ou le trifluorométhyle,
R" représente un alkyle en $C_1$-$C_5$ ou un halogène,
m est égal à 1, 2 ou 3 et n est égal à 0 ou 1,
X représente l'oxygène ou le soufre et
Y' représente l'hydrogène ou $CHF_2$,

y compris leurs sels d'addition d'acides.

2. Agents antiparasitaires pour lutter contre ou prévenir l'infestation des plantes par des nématodes contenant, à côté des supports et adjuvants usuels, comme composant actif, au moins un composé de formule Ib

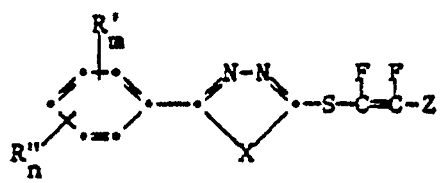

(Ib),

dans laquelle

R' représente un alkyle en $C_1$-$C_5$, un alcoxy en $C_1$-$C_5$, un halogène ou le trifluorométhyle,
R" représente un alkyle en $C_1$-$C_5$ ou un halogène,
m est égal à 1, 2 ou 3 et n est égal à 0 ou 1,
X représente l'oxygène ou le soufre et
Z représente le fluor ou le trifluorométhyle,

y compris leurs sels d'addition d'acides.

3. Agents antiparasitaires pour lutter contre ou prévenir l'infestation des plantes par des nématodes contenant, à côté des supports et adjuvants usuels, comme composant actif, au moins un composé de formule I*

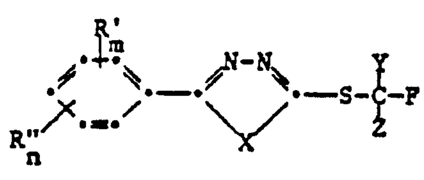

(I*),

dans laquelle

R' représente un alkyle en $C_1$-$C_5$, un alcoxy en $C_1$-$C_5$, un halogène ou le trifluorométhyle,
R" représente un alkyle en $C_1$-$C_5$ ou un halogène,
m est égal à 1, 2 ou 3 et n est égal à 0 ou 1,
X représente l'oxygène ou le soufre,
Y représente l'hydrogène, le fluor, le trifluorométhyle ou le groupe HC(F)Z et
Z représente le fluor ou le trifluorométhyle,

y compris leurs sels d'addition d'acides.

**4.** Agent selon la revendication 1, contenant, comme composant actif, au moins un composé de formule la où R' représente un alkyle en $C_1$-$C_2$, un alcoxy en $C_1$-$C_2$, un halogène ou le trifluorométhyle et R" représente un alkyle en $C_1$-$C_4$ ou un halogène, m est égal à 1 ou 2 et X, Y' et n ont les significations indiquées pour la formule la.

**5.** Agents selon la revendication 1, contenant, comme composant actif, au moins un composé de formule la où R' représente le méthyle, le méthoxy, le fluor ou le chlore et R" représente le fluor ou le chlore, Y' représente l'hydrogène et X et n ont les significations indiquées pour la formule la.

**6.** Agents selon la revendication 2, contenant, comme composant actif, au moins un composé de formule Ib où R' représente un alkyle en $C_1$-$C_2$, un alcoxy en $C_1$-$C_2$, un halogène ou le trifluorométhyle et R" représente un alkyle en $C_1$-$C_4$ ou un halogène, m est égal à 1 ou 2 et X, Z et n ont les significations indiquées pour la formule Ib.

**7.** Agents selon la revendication 2, contenant, comme composant actif, au moins un composé de formule Ib où R' représente le méthyle, le méthoxy, le fluor ou le chlore et R" représente le fluor ou le chlore, Z représente le fluor et X et n ont les significations indiquées pour la formule Ib.

**8.** Agents selon la revendication 3, contenant, comme composant actif, au moins un composé de formule I* où R' représente un alkyle en $C_1$-$C_2$, un alcoxy en $C_1$-$C_2$, un halogène ou le trifluorométhyle et R" représente un alkyle en $C_1$-$C_4$ ou un halogène, m est égal à 1 ou 2 et X, Y, Z et n ont les significations indiquées pour la formule I*.

**9.** Agents selon la revendication 3, contenant, comme composant actif, au moins un composé de formule I* où R' représente le méthyle, le méthoxy, le fluor ou le chlore et R" représente le fluor ou le chlore, Y représente l'hydrogène et Z représente le fluor et X et n ont les significations indiquées pour la formule I*.

**10.** Agents selon la revendication 1 contenant, comme composant actif, au moins un composé de formule la choisi dans le groupe :
2-difluorométhylthio-5-(4'-méthoxyphényl)-1,3,4-oxadiazole;
2-difluorométhylthio-5-(4'-méthoxyphényl)-1,3,4-thiadiazole;
2-difluorométhylthio-5-(2'-fluorophényl)-1,3,4-thiadiazole;
2-difluorométhylthio-5-(4'-chlorophényl)-1,3,4-thiadiazole;
2-difluorométhylthio-5-(2'-méthylphényl)-1,3,4-thiadiazole;
2-difluorométhylthio-5-(2',4'-dichlorophényl)-1,3,4-thiadiazole;
2-difluorométhylthio-5-(2',6'-difluorophényl)-1,3,4-thiadiazole;
2-difluorométhylthio-5-(4'-fluorophényl)-1,3,4-thiadiazole;
2-difluorométhylthio-5-(3'-chlorophényl)-1,3,4-thiadiazole.

**11.** Agents selon l'une des revendications 1 à 10, caractérisé en ce qu'il contient 0,1 à 99 % d'une substance active de formules la, Ib ou I*, 99,9 à 1 % d'un additif solide ou liquide et 0 à 25 % d'un agent de surface.

**12.** Agent selon la revendication 11, caractérisé en ce qu'il contient 0,1 à 95 % d'une substance active de formules la, Ib ou I*, 99,8 à 5 % d'un additif solide ou liquide et 0,1 à 25 % d'un agent de surface.

**13.** Procédé pour la préparation des composés de formules I, Ib ou I* selon les revendications 1-3, caractérisé

a) en ce que l'on fait réagir, dans une réaction de condensation, un composé de formule IIa

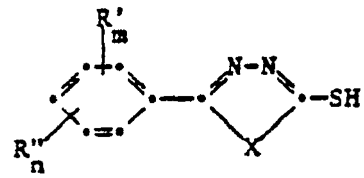

(IIa),

ou un composé de formule IIb

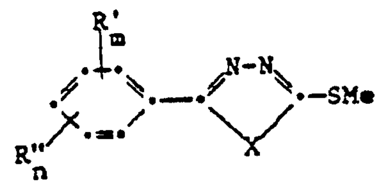

(IIb),

avec un composé de formule IIIa

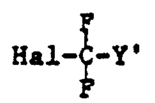

(IIIa)

ou avec un composé de formule III*

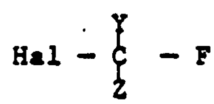

(III*)

dans un solvant ou dans un mélange de solvants inertes à une température augmentée, éventuellement en présence d'un catalyseur et éventuellement à une pression augmentée, la réaction d'un composé de formule IIa s'effectuant, en présence d'une base ou

b) en faisant réagir, dans une réaction d'addition, un composé de formule IIa

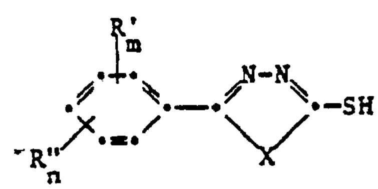

(IIa),

avec un composé de formule IIIb

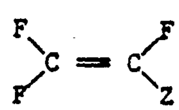

(IIIb)

dans un solvant ou dans un mélange de solvants inertes à une température augmentée, éventuellement en présence d'un catalyseur et éventuellement à une pression augmentée, cette réaction conduisant à un composé de formule I*

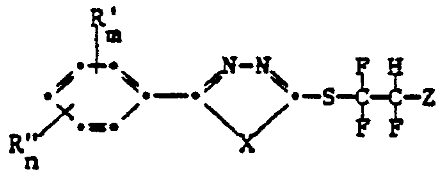

(Ia*),

ou à un composé de formule Ib

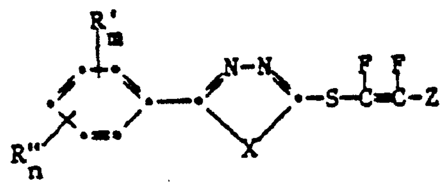

(Ib),

dans les formules IIa, IIb, Ia*, Ib, IIIa, IIIb et III* précédemment citées, Me désigne un métal alcalin ou l'ammonium, Hal un halogène, de préférence le chlore, le brome ou l'iode, tandis que R', R", m, n, Y, Y' et Z ont les significations indiquées pour les formules Ia, Ib ou I*.

**14.** Procédé pour la préparation d'un agent agrochimique, caractérisé en ce que l'on mélange intimement au moins un composé de formules Ia, Ib ou I* selon l'une des revendications 1 à 3, avec des additifs solides ou liquides appropriés et des agents de surface.

**15.** Procédé pour lutter contre ou prévenir une infestation des plantes cultivées par des nématodes, caractérisé en ce que l'on applique un composé de formules Ia, Ib ou I* selon les revendications 1 à 3 sur la plante ou sur son emplacement.

**16.** Utilisation des composés de formules Ia, Ib ou I* selon les revendications 1 à 3 pour lutter contre et/ou prévenir l'infestation des plantes par des nématodes.

**17.** Utilisation selon la revendication 16, caractérisée en ce que les nématodes sont des espèces parasitant les racines des plantes.

**18.** Utilisation contre les nématodes du genre Meloidogyne, Heterodera ou Globodera, selon la revendication 17.